(19) Europäisches Patentamt
European Patent Office
Office européen des brevets

(11) Publication number: 0 058 015
A2

# (12) EUROPEAN PATENT APPLICATION

(21) Application number: 82300399.1

(22) Date of filing: 27.01.82

(51) Int. Cl.$^3$: A 61 K 31/495
A 61 K 31/43, A 61 K 9/10
A 61 M 5/24
//(A61K31/43, 31/495)

(30) Priority: 05.02.81 GB 8103583

(43) Date of publication of application: 18.08.82 Bulletin 82/33

(84) Designated Contracting States: BE CH DE FR GB IT LI NL

(71) Applicant: BEECHAM GROUP PLC
Beecham House Great West Road
Brentford Middlesex(GB)

(72) Inventor: Craven, Neil
37 Beedon Hill
Newbury Berkshire(GB)

(72) Inventor: Anderson, James Currie
17 Goldwell Drive
Newbury Berkshire(GB)

(74) Representative: Cresswell, Thomas Anthony et al,
European Patent Attorney Beecham Pharmaceuticals
Great Burgh Yew Tree Bottom Road
Epsom Surrey KT18 5XQ(GB)

## (54) Treatment of infections.

(57) Mastitis, especially bovine mastitis, is treated by intramammary administration of isoxazolyl penicillin and rifampicin.

EP 0 058 015 A2

## TREATMENT OF INFECTIONS

This invention relates to a method for treating mastitis in mammals, and especially, but not exclusively, to a method for treating bovine mastitis.

It is known to use antibiotics in the treatment and control of mastitis.

In particular, it is known to use semi-synthetic penicillins, such as isoxazolyl penicillins, in the treatment of mammary disorders. Suitable veterinary compositions for this type of treatment are disclosed in U K Patent Specification No 1547164.

One disadvantage of the conventional use of an isoxazolyl penicillin in treating mastitis is that, in cases of mastitis, the staphylococci responsible for the disease are mostly intracellular and non-dividing and hence are refractory to the penicillin.

It has now surprisingly been found that these intracellular staphylococci can be substantially eliminated by treatment with an isoxazolyl penicillin and rifampicin.

Previously, there had been no suggestion that rifampicin could be used in treating mastitis. In bovine mastitis, therapy with another member of the rifamycin group, rifamycin-S.V., has been tried, but recent reports have suggested that the treatment is less effective than penicillin based preparations.

[Poutrel, B.(1978) Annales de Recherches Veterinaires 9, 471-487.

Postle, D.S., Roguinsky, M. and Poutrel,B. (1979) American Journal of Veterinary Research 40, 618-622]

Combinations of penicillins and rifampicin have been tested for synergy or antagonism in multiple antibiotic therapy, using *in vitro* testing against four strains of *Staphylococcus aureus* but no evidence of synergy or antagonism has been detected [Sanderson P J and Drabu Y J Journal of Antimicrobial Chemotherapy (1979) 5, 728-730]. Furthermore, there was no indication in the latter publication that a combination of isoxazolyl penicillin and rifampicin would have any value in the treatment of mastitis.

It is believed that the surprising advantage shown by the combination of isoxazolyl penicillin and rifampicin is due to the fact that the penicillin prevents the development of bacteria having rifampicin resistance, while the rifampicin itself is able to kill intracellular staphylococci. In the absence of the penicillin, rifampicin would be ineffective in the treatment of mastitis because of the gradual emergence of rifampicin resistant organisms. In addition to preventing the development

of rifampicin resistance, the penicillin is able to kill extracellular and dividing bacteria, so that the combination of the two active ingredients can attack both intracellular and extracellular bacteria.

In its broadest aspect, therefore, the invention provides a method for treating mastitis in mammals, which comprises administering to a mammal by the intramammary route effective amounts of an isoxazolyl penicillin and rifampicin. The method is especially suited to the treatment of mastitis in cattle.

Preferably the penicillin and rifampicin are administered together in combination with a veterinarily acceptable carrier.

Preferred isoxazolyl penicillins are cloxacillin and flucloxacillin, and they may be conveniently used in the form of their lipid-soluble esters, which have better tissue distribution and penetration than the penicillins themselves.

The active ingredients may be incorporated into any suitable veterinary carrier for intramammary administration. For example, the ingredients may be suspended in an oily vehicle, with an appropriate thickening agent.

For such administration, the chosen suspension may be filled into tubes or syringe packs of the conventional type for intramammary adminstration, i.e. provided with a cannula nozzle for insertion into the teat to allow extrusion directly into the mammary gland *via* the streak canal.

For the treatment of bovine mastitis, a single dose will normally contain 1 to 10 gm., preferably 3 to 8 gm., of the suspension. The weights of penicillin and rifampicin in such a unit dose will of course depend on the severity of the disorder to be treated and can vary within wide limits. An effective treatment can usually be obtained with a weight of penicillin in the range of about 100 to 1000 mg preferably about 200 mg, per unit dose, and a weight of rifampicin in the range of about 25 to 500 mg preferably about 200 to 300mg, per unit dose. For example, a dose containing about 200 mg of cloxacillin and about 300 mg of rifampicin has been found to be particularly effective in the treatment of bovine mastitis.

Often a single dose of the composition of the invention will provide effective treatment of the mammary disorder. However, it is usual practice to repeat the dose at least once (preferably three doses are given), each dosing taking place after milking.

In treating bovine mastitis, where numbers of staphylococci in the udder are sufficiently high to make the emergence of rifampicin resistant cocci likely, it may be desirable to provide cloxacillin cover before and after exposure to rifampicin. Exposure to cloxacillin first would effectively reduce replicating S. aureus and thus also decrease the risk of subsequent induction of rifampicin resistance.

This therapeutic strategy could be incorporated into a standard regime of three intramammary infusions at 12 or 24 hour intervals, by treating first with

cloxacillin, then with a combination of cloxacillin and rifampicin, and finally with cloxacillin alone.

This three stage treatment provides a further aspect of the present invention.

Isoxazolyl penicillins may be produced by any conventional process, such as that described in UK Patents No. 905 778 and 978 299 which are incorporated herein by reference. A particularly preferred process is to react the acid chloride, bromide, anhydride or mixed anhydride derived from a compound of formula

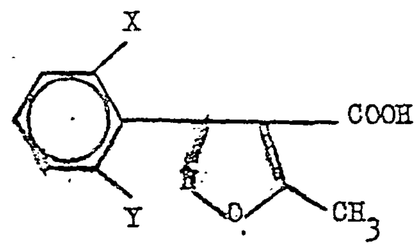

wherein X and Y are the same or different and each is chlorine, bromine or fluorine

with 6-aminopenicillanic acid or a liquor containing 6-aminopenicillanic acid, as described in UK Patent No. 978 299.

The following Examples illustrate compositions which may be used in the method of the invention for treating bovine mastitis.

Example 1

1 kg of a veterinary preparation for lactating cows was prepared according to the following composition:

| | | % by wt. |
|---|---|---|
| Sodium cloxacillin | | 6.7 (as free acid) |
| Rifampicin | | 6.7 |
| 12-hydroxystearin | | 0.75 |
| Polysorbate 80 | | 0.125 |
| Sorbitan Mono-oleate | | 0.125 |
| Butylated hydroxyanisole | | 0.02 |
| Fractionated Coconut Oil | to | 100 |

The 12-hydroxystearin, polysorbate 80, sorbitan mono-oleate and butylated hydroxyanisole were dissolved in the required amount of fractionated coconut oil. The mixture was sterilised by heating to 150°C and maintaining this temperature for one hour and then allowed to cool.

The required amounts of sodium cloxacillin and rifampicin were incorporated into the base by high shear stirring.

The suspension was filled as 3g doses into intramammary syringes.

Example 2

1 kg of an intramammary product for dry cows was prepared according to the following composition:

| | | % by wt. |
|---|---|---|
| Benzathine cloxacillin equivalent to cloxacillin | | 16.7 |
| Rifampicin | | 16.7 |
| 12-hydroxystearin | | 4.0 |
| Colloidal silica | | 1.0 |
| Butylated hydroxyanisole | | 0.02 |
| Arachis oil | to | 100 |

The arachis oil was mixed with the butylated hydroxyanisole, colloidal silica and 12-hydroxystearin. The mixture was sterilised by heating to 150°C and the temperature held for one hour and the mixture then allowed to cool. The benzathine cloxacillin and rifampicin were incorporated into the base by high shear stirring.

The suspension was filled into intramammary syringes at a dose of 3g.

1. Experimental Mouse Data

(A) Cloxacillin/Rifampicin Treatment

Lactating mice of the Compton white strain were used 4-7 days after parturition. The mice were anaesthetised with ether and c.$10^6$ cfu S. aureus were inoculated via the teat into the fourth mammary gland on each side (R4 and L4) by the method described by Anderson [Zentralblatt fur Bakteriologie, Parasitenkunde, Infektionskrankheiten und Hygiene (Abteilung 1) Suppl. 5 783-790].

4 Hours later, the mice were divided into four groups and each group received inoculations into R4 and L4 of either cloxacillin, rifampicin, cloxacillin and rifampicin, or saline. After a further 18 hours, the mice were killed. From each mouse, L4 was removed and homogenised in a Griffith's tube with 5 ml sterile saline, and a total viable bacterial count was determined using ox blood agar plates pretreated with β-lactamase.

Statistical Analysis

The $\log_{10}$ bacterial viable counts per gland found after antibiotic (or saline) therapy in each experiment were examined by analysis of variance. Differences between each pair of treatments were then compared for significance in Student's 't' tests, using the value of the mean square for error obtained in the analysis of variance.
Antibiotic residues. Each gland homogenate was also assayed for antibiotic content using an agar well diffusion method with penassay seed agar (Antibiotic medium 1, Difco Labs., Detroit) and Sarcina lutea as the test organism.

Rifampicin was assayed in the presence of cloxacillin by adding 2 iu of β-lactamase to 1 ml homogenate and incubating at 37°C for 3o mins to inactivate the cloxacillin, before adding it to the wells. The cloxacillin activity was estimated from untreated homogenate.

RESULTS

The mean viable bacteria per gland ($\log_{10}$ cfu/gland) for groups of five mice with acute mastitis and the effects of therapy with rifampicin, cloxacillin or both are shown in Fig. 1. 4 hours after inoculating c.2 x $10^6$ cfu M60 S. aureus the mice were clinically normal. The control group of five mice killed at this time showed that bacterial multiplication was occuring, as the geometric mean viable count per gland was now 7.13 x $10^7$ cfu. After a further 18 hours, of the five mice inoculated with saline only, four were dead and the fifth was moribund. The glands were necrotic and a mean of 5.81 x $10^9$ viable cfu of M60 per gland were recovered. All of the antibiotic treated mice were clinically normal. The mean viable bacterial counts per gland obtained from all the antibiotic treated groups of mice were significantly much lower than the control group ($P<0.01$). Mice treated with the combination of rifampicin with cloxacillin had significantly lower counts than those treated with either antibiotic alone ($P<0.01$). Cloxacillin alone reduced bacterial counts to a significantly greater extend than rifampicin treatment ($P<0.01$).

Staphylococci recovered from three of the five mice treated with rifampicin alone were rifampicin resistant (MIC>6.3 μg/ml). S. aureus recovered from all the other mice remained sensitive (MIC 0.02 μg/ml rifampicin).

(B) Flucloxacillin/Rifampicin Treatment

Similar treatment to that described above was carried out on mice, in which cloxacillin was replaced by flucloxacillin.

The results are shown in Table 1 and clearly show that a combination of flucloxacillin and rifampicin reduce bacterial counts to a greater extent than either antibiotic alone.

TABLE 1

| 4 h bacterial counts ($log_{10}$ cfu/gland) (at time of therapy) | | 22 h bacterial counts ($log_{10}$ cfu/gland) (18 h after treatment with:-) Saline | Fluclox (1 mg) | Rifampicin (25 μg) | Flu (1 mg) + Rif (25 μg) |
|---|---|---|---|---|---|
| | 7.544 | 9.980 | 4.243 | 5.694 | 3.118 |
| | 7.588 | 9.740 | 4.796 | 4.581 | 2.641 |
| | 7.528 | 9.792 | 4.158 | 5.536 | 3.459 |
| | 7.536 | 10.012 | 4.340 | 4.258 | 4.211 |
| | | 9.364 | 4.477 | 4.118 | 3.287 |
| $\bar{x}$ | 7.549 | 9.778 | 4.403 | 4.837 | 3.343 |
| S.E.M. | 0.013 | 0.116 | 0.112 | 0.327 | 0.256 |

Fig. 1 Therapy of acute staphylococcal mastitis in mice

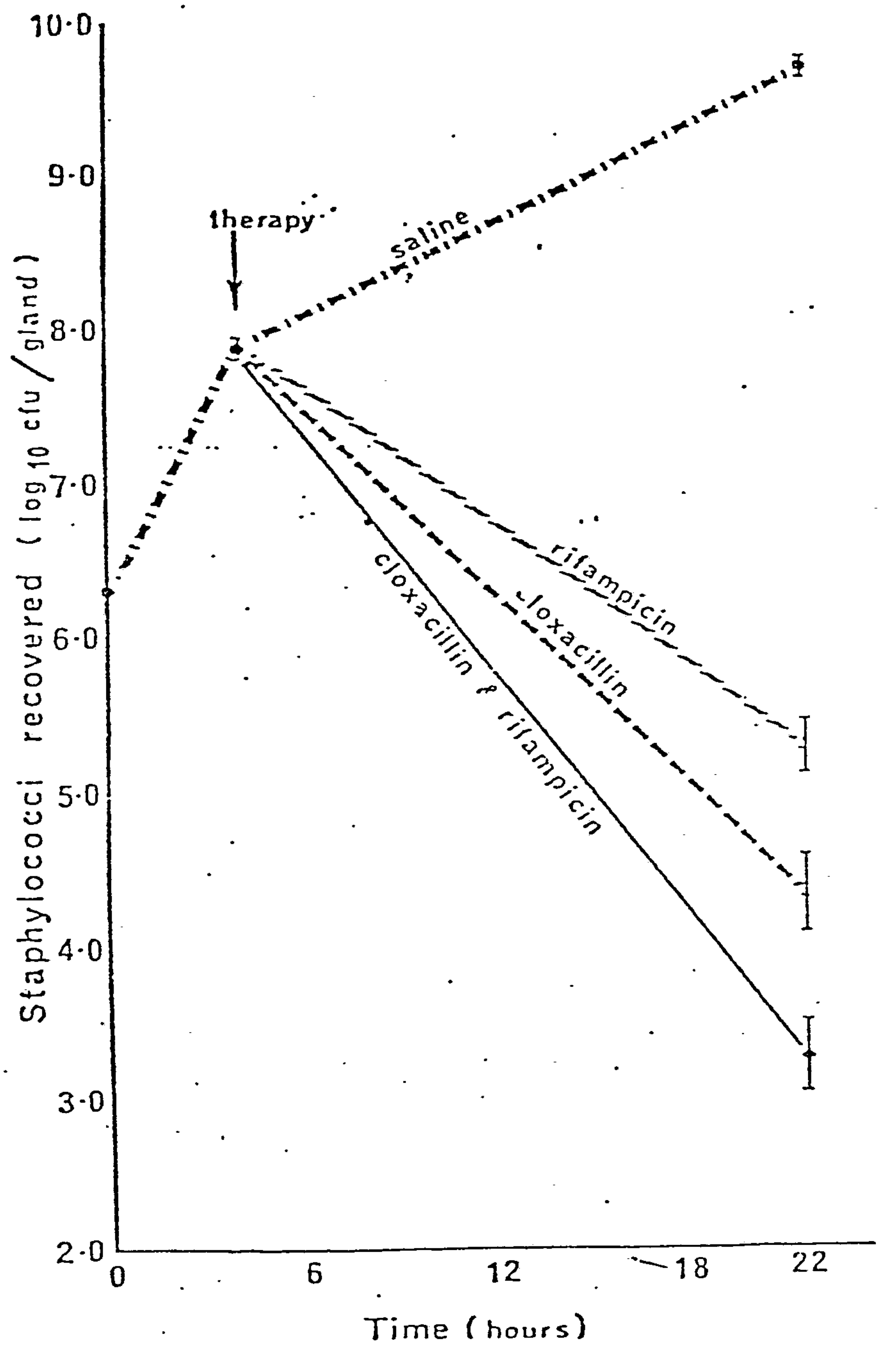


Antibiotics inoculated into mammary glands 4 h after infection at a dose of 1 mg cloxacillin or 25 g rifampici per gland, or both. Each point shows the mean viable bacterial count per gland for groups of five mice ± standa error or mean.

BOVINE MASTITIS DATA

Twenty one staphylococcal mastitis infections were treated as follows:

Ten infections treated with 3 x 12 hr infusions of a combination of 300 mg Rifampicin and 200 mg Cloxacillin.

Eleven infections treated with 3 x 12 hr infusions of 200 mg Cloxacillin.

The treatment was carried out by intramammary infusion in lactating cows, and the clinical and bacteriological response recorded at 4 and 7 days after treatment.

Of the ten quarters treated with the rifampicin/cloxacillin combination, only two showed clinical abnormalities (i.e. clots or firmness) and only four had raised milk cell counts.

Four days after treatment, all but one quarter were clear of pathogens, and in three of the four raised milk cell counts, the counts had declined. The condition of the two clinical cases was unchanged. Seven days after treatment, two out of the ten quarters yield staphylococci, while the condition of one clinical case had improved.

Infections treated with cloxacillin alone included eight out of eleven with raised cell counts and four out of eleven with clinical symptoms of mastitis.

Four days after treatment, five quarters yielded staphylococci and five had raised cell counts. Seven days after treatment, six quarters yielded staphylococci.

The results can be summarised in the following table:

| Treatment | Proportion of quarters cured four days after treatment | Proportion of quarters cured seven days after treatment |
| --- | --- | --- |
| Rifampicin + Cloxacillin | 9 out of 10 | 8 out of 10 |
| Cloxacillin | 6 out of 11 | 5 out of 11 |

The above results clearly show the superior efficacy of the combination over cloxacillin alone at four and seven days after treatment.

CLAIMS

1. A veterinary formulation for intramammary administration comprising an isoxazolyl penicillin and rifampicin in association with a veterinarily acceptable carrier therefor.

2. A formulation as claimed in claim 1 wherein the isoxazolyl penicillin is cloxacillin or flucloxacillin.

3. A formulation as claimed in claim 1 or 2 presented in unit dosage form.

4. A formulation as claimed in claim 3 comprising from 100 to 1000 mg of the isoxazolyl penicillin per unit dose.

5. A formulation as claimed in claim 4 comprising about 200 mg of isoxazolyl penicillin per unit dose.

6. A formulation as claimed in any one of claims 3 to 5 comprising about 25 to 500 mg of rifampicin per unit dose.

7. A formulation as claimed in claim 6 comprising about 200 to 300 mg of rifampicin per unit dose.

8. A syringe for intramammary administration of medicament having a cannula nozzle and containing a formulation as claimed in any one of claims 1 to 7.

9. A process for producing a formulation as claimed in any one of claims 1 to 7 which process comprises bringing into association the isoxazolyl penicillin, rifampicin and carrier therefor.